(19) Europäisches Patentamt
European Patent Office
Office européen des brevets

(11) EP 0 943 369 A1

(12) **EUROPÄISCHE PATENTANMELDUNG**

(43) Veröffentlichungstag:
**22.09.1999 Patentblatt 1999/38**

(51) Int. Cl.$^6$: **B01F 5/10**

(21) Anmeldenummer: **99104453.8**

(22) Anmeldetag: **05.03.1999**

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **07.03.1998 DE 19809945**

(71) Anmelder:
**Fresenius Medical Deutschland GmbH**
**61350 Bad Homburg v.d.H. (DE)**

(72) Erfinder:
- **Christmann-Braun, Horst**
  **61440 Oberursel (DE)**
- **Koerdt, Franz-Wilhelm**
  **61231 Bad Nauheim (DE)**
- **Metzner, Klaus**
  **61381 Friedrichsdorf (DE)**

(74) Vertreter:
**Luderschmidt, Schüler & Partner GbR**
**Patentanwälte,**
**John-F.-Kennedy-Strasse 4**
**65189 Wiesbaden (DE)**

(54) **Einrichtung zur Bereitstellung von Dialysierflüssigkeit mit einer Einrichtung zur Überwachung ausgewählter Parameter der Dialysierflüssigkeit und Verfahren zur Überwachung ausgewählter Parameter der Dialysierflüssigkeit bei einer Dialysebehandlung**

(57) Zur Überwachung ausgewählter Parameter der Dialysierflüssigkeit, insbesondere deren Zusammensetzung oder der Dialysierflüssigkeitsfluß, wird die Ausbreitungsgeschwindigkeit von Ultraschallimpulsen in der Dialysierflüssigkeit gemessen. Die Konzentration der in der Dialysierflüssigkeit enthaltenen Substanzen kann somit unabhängig von deren Leitfähigkeit bestimmt werden, so daß sich beispielsweise auch die Konzentration von Glucose in der Dialysierflüssigkeit erfassen läßt.

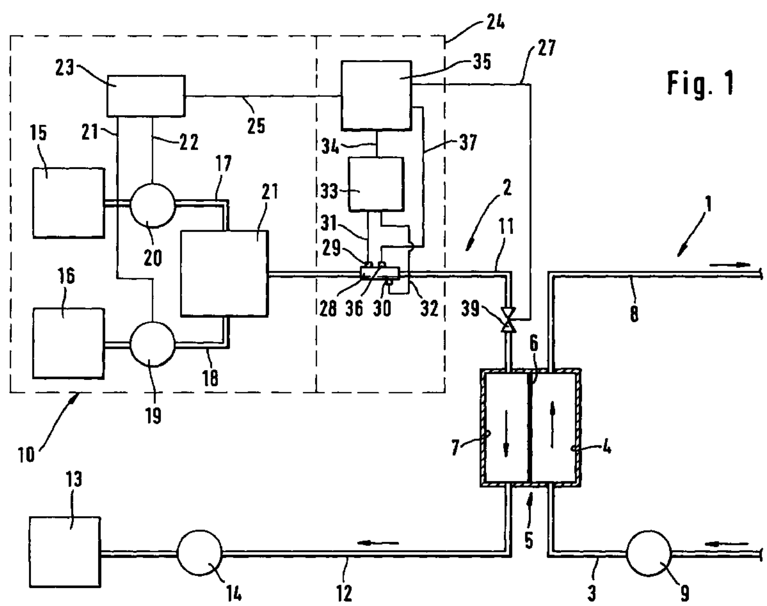

Fig. 1

EP 0 943 369 A1

## Beschreibung

Die Erfindung betrifft eine Einrichtung zur Bereitstellung von Dialysierflüssigkeit, die eine Einrichtung zur Überwachung ausgewählter Parameter der Dialysierflüssigkeit aufweist. Darüber hinaus bezieht sich die Erfindung aufein Verfahren zur Überwachung ausgewählter Parameter der Dialysierflüssigkeit bei einer Dialysebehandlung.

Die bekannten Hämodialysevorrichtungen weisen eine Einrichtung zur Bereitstellung der Dialysierflüssigkeit auf. Die Dialysierflüssigkeit wird im allgemeinen aus einer abgemessenen Menge von flüssigem oder pulverförmigem Konzentrat und einer vorgegebenen Menge von Wasser in der gewünschten Konzentration gemischt. Hierzu finden beispielsweise motorisch angetriebene Proportionierungspumpen Verwendung, bei denen weitgehend unabhängig von äußeren Betriebsbedingungen ein gleichbleibender Zusammenhang zwischen der Zahl der Arbeitszyklen und dem dabei geförderten Volumen besteht. Es sind aber auch Einrichtungen zur Bereitstellung von Dialysierflüssigkeit bekannt, die ein mit Füllstandssonden ausgestattetes Meßgefäß aufweisen, das in aufeinanderfolgenden Arbeitszyklen jeweils bis zu einem bestimmten Füllstand abwechselnd mit Wasser oder Dialysekonzentrat befüllt wird.

Die Bereitstellung der Dialysierflüssigkeit kann bei gleichzeitigem Betrieb mehrerer Dialysevorrichtungen in einer zentralen Versorgungseinrichtung erfolgen. Die Einrichtung zur Bereitstellung von Dialysierflüssigkeit kann aber auch Bestandteil der Dialysevorrichtung sein.

Zur Überwachung der Konzentration der Dialysierflüssigkeit verfügen die bekannten Einrichtungen zur Bereitstellung von Dialysierflüssigkeit über eine entsprechende Überwachungseinrichtung. Bei bekannten Dialysevorrichtungen wird mit einer derartigen Überwachungseinrichtung die Zufuhr von Dialysierflüssigkeit in den Dialysator unterbrochen, wenn die Dialysierflüssigkeitszusammensetzung von einem Sollwert abweicht. Die Überwachung der Dialysierflüssigkeitszusammensetzung erlaubt aber auch die Steuerung der Proportionierungspumpen, um die korrekte Dialysierflüssigkeitskonzentration einzustellen.

Alle bisher bekannten Verfahren zur Überwachung der Zusammensetzung der Dialysierflüssigkeit beruhen auf einer Leitfähigkeitsmessung. Hämodialysegeräte mit einer derartigen Leitfähigkeitsmeßeinrichtung zur Überwachung der Zusammensetzung der Dialysierflüssigkeit sind beispielsweise aus der EP-A-0 311 848 und EP-A-0 443 324 bekannt.

Die elektrische Leitfähigkeit einer wässrigen Lösung ist an das Vorhandensein von Ionen geknüpft, die hauptsächlich von den gelösten Salzen gebildet werden. Der Beitrag der einzelnen Ionenarten zur Gesamtleitfähigkeit hängt von der Konzentration und von der Beweglichkeit der jeweiligen Ionenart ab. Ionen mit hoher Beweglichkeit, z.B. Chlorid, leisten einen höheren Beitrag, während solche mit niedrigerer Beweglichkeit, z.B. Acetat, bei gleicher Konzentration einen niedrigeren Beitrag zur Gesamtleitfähigkeit leisten.

Gelöste Stoffe, die keine oder nur wenig Ionen bilden, liefern keinen Beitrag, z.B. Glucose, oder nur einen sehr geringen Beitrag zur elektrischen Leitfähigkeit, z.B. Formalin. Die Aussagefähigkeit der elektrischen Leitfähigkeit für die Zusammensetzung der Dialysierflüssigkeit ist somit stark eingeschränkt. So können beispielsweise schädliche Beimengungen, z.B. Formalin, im allgemeinen nicht erkannt werden. (Dialysetechnik, Dieter Schleipfer, 4. überarbeitete Auflage, Gesellschaft für angewandte Medizintechnik mbH & Co. KG, Friedrichsdorf, 1988, Seiten 212 f.).

Neben der Konzentration einer bestimmten Substanz in der Dialysierflüssigkeit ist als weiterer Parameter der Dialysierflüssigkeit auch deren Flußrate von Interesse. In der Dialysierflüssigkeitsaufbereitung wird bei den bekannten Dialysemaschinen im allgemeinen auf eine eigene Durchflußsensorik verzichtet, wenn die Flußrate durch die Fördermenge der Pumpen vorgegeben wird.

Die US-A-4,015,470 beschreibt eine Vorrichtung und ein Verfahren zur Bestimmung gewünschter Parameter einer Flüssigkeit. Die bekannte Vorrichtung und das bekannte Verfahren beruhen auf einer Messung der Laufzeit von Schallimpulsen in der Flüssigkeit. Eine derartige Laufzeitbestimmung ist aber bisher bei Dialysevorrichtungen zur Bestimmung ausgewählter Parameter der Dialysierflüssigkeit nicht eingesetzt worden, obwohl die zugrundeliegende Technik seit Jahrzehnten bekannt war.

Der Erfindung liegt die Aufgabe zugrunde, eine Einrichtung zur Bereitstellung von Dialysierflüssigkeit für eine Dialysevorrichtung zu schaffen, bei der eine Überwachung ausgewählter Parameter der Dialysierflüssigkeit, insbesondere deren Zusammensetzung oder der Dialysierflüssigkeitsfluß, auch dann mit großer Genauigkeit erfolgt, wenn die Dialysierflüssigkeit Substanzen mit nur geringer Leitfähigkeit enthält. Die Lösung dieser Aufgabe erfolgt mit den im Patentanspruch 1 angegebenen Merkmalen.

Eine weitere Aufgabe der Erfindung liegt darin, ein Verfahren anzugeben, das die Überwachung ausgewählter Parameter der Dialysierflüssigkeit auch dann mit großer Genauigkeit erlaubt, wenn die Dialysierflüssigkeit Substanzen mit nur geringer Leitfähigkeit enthält. Diese Aufgabe wird mit den Merkmalen des Patentanspruchs 7 gelöst.

Die Bestimmung der ausgewählten Parameter der Dialysierflüssigkeit, insbesondere deren Zusammensetzung, basiert nicht auf einer Leitfähigkeitsmessung, sondern auf der Ermittlung der Ausbreitungsgeschwindigkeit von Ultraschallimpulsen in der Dialysierflüssigkeit. Diese Ausbreitungsgeschwindigkeit, die durch die Messung der Laufzeit der Ultraschallimpulse über eine Meßstrecke mit einer vorgegebenen Länge bestimmt wird, ist abhängig von der Dichte der Flüssigkeit. Da die Konzentration einer in der Dialysierflüssigkeit enthaltenen Substanz Einfluß auf deren Dichte hat, kann die Konzentration der Substanz in der Dialysierflüssigkeit mit der Laufzeitmessung unabhängig von der Leitfähigkeit mit großer Genauigkeit bestimmt werden. Von Vorteil ist auch, daß die Mittel zum Senden und Empfangen der Ultraschallimpulse nicht in direktem Kontakt mit der Dialysierflüssigkeit stehen müssen. So können die Mittel zum Senden und Empfangen der Ultraschallimpulse beispielsweise an der Außenseite eines Behälters angeordnet sein, der die frische Dialysierflüssigkeit aufnimmt. Vorzugsweise sind die Mittel aber an der Außenseite einer Meßleitung angeordnet, durch die die Dialysierflüssigkeit geleitet wird.

Die Ermittlung der Ausbreitungsgeschwindigkeit von Ultraschallimpulsen erlaubt bei einer verschiedene Stoffe enthaltenden Lösung die Überwachung deren Gesamtdichte. Wenn die Dialysierflüssigkeit durch Mischen von Wasser mit mehreren Konzentraten hergestellt wird, kann die Konzentration jedes gelösten Stoffes in der jeweiligen Mischleitung bzw. im Mischbehälter bestimmt werden. Hierzu sind dann mehrere Meßanordnungen, die jeweils über Mittel zum Senden und Empfangen der Ultraschallimpulse verfügen, erforderlich. Wenn das Mischungsverhältnis der einzelnen Konzentrate bekannt ist, können dann die absoluten Konzentrationen bestimmt werden.

Der Vorteil der Ultraschallmessung liegt darin, daß in einer Lösung auch Stoffe erfaßt werden können, die nicht oder nur geringfügig zur elektrischen Leitfähigkeit beitragen.

Bei einem bevorzugten Ausführungsbeispiel werden Ultraschallimpulse zur Ermittlung einer ersten Impulslaufzeit in Strömungsrichtung der durch die Meßleitung geleiteten Dialysierflüssigkeit an einem Ende einer quer zur Längsachse der Meßleitung verlaufenden Meßstrecke erzeugt und an dem anderen Ende der Meßstrecke empfangen, während zur Ermittlung einer zweiten Impulslaufzeit entgegen der Strömungsrichtung der Dialysierflüssigkeit Ultraschallimpulse an dem anderen Ende der Meßstrecke erzeugt und an dem einen Ende der Meßstrecke empfangen werden. Hierzu umfassen die Mittel zum Senden und die Mittel zum Empfangen der Ultraschallimpulse zweckmäßigerweise einen ersten und einen zweiten Ultraschallwandler, von denen der erste an dem einen und der zweite an dem anderen Ende der Meßstrecke angeordnet ist. Aus der ersten und zweiten Impulslaufzeit kann als ausgewählter Parameter der Dialysierflüssigkeit neben deren Zusammensetzung auch der Dialysierflüssigkeitsfluß bestimmt werden. Dies ist insofern vorteilhaft, als separate Durchflußmesser, die eine aufwendige Meßtechrik erfordern, sofern diese berührungslos arbeiten, nicht mehr erforderlich sind.

Bei einer Ausführungsform, die nur die Bestimmung der Dichte, nicht aber des Dialysierflüssigkeitsflusses als hämodynamischer Parameter erlaubt, verläuft die Meßstrecke im rechten Winkel zur Längsachse der Meßleitung. Die Überwachungseinheit verfügt über einen Ultraschallwandler zum Senden und einen Ultraschallwandler zum Empfangen der Ultraschallimpulse. Die Dichte wird aus der Impulslaufzeit längs der Meßstrecke, bzw. der Ausbreitungsgeschwindigkeit der Ultraschallimpulse, bestimmt.

Da die Ausbreitungsgeschwindigkeit der Ultraschallimpulse in der Dialysierflüssigkeit temperaturabhängig ist, erfolgt vorteilhafterweise eine Temperaturkompensation.

Das erfindungsgemäße Verfahren und die erfindungsgemäße Vorrichtung sind vorzugsweise zur Verwendung in Hämodialysevorrichtungen und Vorrichtungen für die Peritonialdialyse bestimmt.

Im folgenden wird ein Ausführungsbeispiel der Erfindung unter Bezugnahme auf die Zeichnungen näher erläutert.

Es zeigen:

Figur 1 eine Prinzipskizze einer Hämodialysevorrichtung mit einer Einrichtung zur Bereitstellung der Dialysierflüssigkeit, die eine Einrichtung zur Überwachung der Zusammensetzung und des Dialysierflüssigkeitsflusses aufweist,

Figur 2 eine Prinzipskizze zur Erläuterung des Verfahrens zur Überwachung der Zusammensetzung und des Dialysierflüssigkeitsflusses und

Figur 3 die Ausbreitungsgeschwindigkeit der Ultraschallimpulse in der Dialysierflüssigkeit als Funktion des Volumens bzw. der Menge von verschiedenen Substanzen, die der Dialysierflüssigkeit hinzugefügt wurden.

Figur 1 zeigt die wesentlichen Baugruppen einer Hämodialysevorrichtung, die über eine Einrichtung zur Bereitstellung frischer Dialysierflüssigkeit mit einer Einrichtung zur Überwachung der Zusammensetzung und des Dialysierflüssigkeitsflusses verfügt. Die Hämodialysevorrichtung umfaßt einen extrakorporalen Blutkreislauf 1 und einen Dialysierflüssigkeitskreis 2. Der extrakorporale Blutkreislauf 1 weist eine arterielle Blutleitung 3 auf, die von dem Patienten zu dem Einlaß einer Blutkammer 4 eines Dialysators 5 führt, der durch eine semipermeable Membran 6 in die Blutkammer 4 und eine Dialysierflüssigkeitskammer 7 geteilt ist. Von dem Auslaß der Blutkammer 4 des Dialysators 5 führt eine venöse Blutleitung 8 zurück zum Patienten. In die arterielle Blutleitung 3 ist eine Blutpumpe 9 geschaltet.

Die Einrichtung 10 zur Bereitstellung frischer Dialysierflüssigkeit ist über eine Dialysierflüssigkeitszuführleitung 11 mit dem Einlaß der Dialysierflüssigkeitskammer 7 des Dialysators 5 verbunden und der Auslaß der Dialysierflüssigkeitskammer ist über eine Dialysierflüssigkeitsabführleitung 12 mit einem Auslauf 13 verbunden. In die Dialysierflüssigkeitszuführleitung 11 ist stromauf des Dialysators 5 ein elektromagnetisch betätigbares Sperrventil 39 geschaltet und in die Dialysierflüssigkeitsabführleitung 12 ist stromab des Dialysators eine Dialysierflüssigkeitspumpe 14 geschaltet.

Beim Betrieb der Dialysevorrichtung wird frische Dialysierflüssigkeit mittels der Dialysierflüssigkeitspumpe 14 durch die Dialysierflüssigkeitskammer des Dialysators gepumpt, während mittels der Blutpumpe 9 Blut des Patienten durch die Blutkammer gepumpt wird.

Die Einrichtung 10 zur Bereitstellung der frischen Dialysierflüssigkeit weist eine Dialysierflüssigkeitsquelle auf, die eine Wasserquelle 15 und eine Dialysierflüssigkeitskonzentratquelle 16 umfaßt, die über Zuführleitungen 17, 18, in die jeweils eine Proportionierungspumpe 19, 20 geschaltet sind, mit einem Mischbehälter 21 verbunden sind, an dessen Auslaß die Dialysierflüssigkeitszuführleitung 11 angeschlossen ist. Die Förderraten der Proportionierungspumpen 19, 20 werden über Steuerleitungen 21, 22 von einer Steuereinheit 23 vorgegeben, die das gewünschte Mischungsverhältnis von Wasser und Konzentrat einstellt.

Die Zusammensetzung und die Flußrate der Dialysierflüssigkeit wird mittels der Überwachungseinrichtung 24, die über eine Datenleitung 25 mit der Steuereinheit 23 der Einrichtung 10 zur Bereitstellung frischer Dialysierflüssigkeit und über eine Datenleitung 27 mit dem Absperrventil 12 verbunden ist, laufend überwacht.

Wenn die Zusammensetzung der Dialysierflüssigkeit von ihrem Sollwert so stark abweicht, daß der Patient gefährdet werden könnte, schließt die Überwachungseinrichtung das Sperrventil 12 zur Unterbrechung der Dialysierflüssigkeitszuführ in den Dialysator 5. Darüber hinaus liefert die Überwachungseinrichtung die erforderlichen Daten für die Steuereinheit 23 zur Einstellung des gewünschten Mischungsverhältnisses. Wenn das Mischungsverhältnis von dem Sollwert abweicht, wird die Abweichung von der Überwachungseirrichtung 24 erfaßt. Die Steuereinheit 23 korrigiert dann die Förderraten der Proportionierungspumpen 19, 20.

Nachfolgend wird die Funktionsweise der Überwachungseinrichtung im einzelnen erläutert.

Die Überwachungseinrichtung 24 weist ein in die Dialysierflüssigkeitszuführleitung 11 stromauf des Dialysators 5 geschaltetes Meßrohr 28 auf. An der Außenseite des Meßrohrs 28 befinden sich zwei Ultraschallwandler 29, 30, mit denen jeweils Ultraschallsignale gesendet oder empfangen werden können (Figur 2). Über Meßleitungen 31, 32 sind die Ultraschallwandler 29, 30 mit einer Einrichtung 33 zum Umschalten zwischen Sende- und Empfangsbetrieb verbunden, die über eine Datenleitung 34 an einer Rechen- und Auswerteinheit 35 angeschlossen ist. Ferner ist ein die Temperatur der Dialysierflüssigkeit erfassender Temperatursensor 36 vorgesehen, der über eine Datenleitung 37 mit der Rechen- und Auswerteinheit 35 verbunden ist.

Die Ultraschallwandler 29, 30 haben eine gemeinsame Sende- bzw. Empfangsachse 38, d.h. die Richtung in der Ultraschallimpulse gesendet bzw. empfangen werden. Die Wandler 29, 30 sind derart an der Außenseite des Meßrohrs 28 angeordnet, daß deren Sende- bzw. Empfangsachse 38 unter einem Winkel $\alpha$ schräg zur Längsachse des Meßrohrs und damit zur Strömungsrichtung w der Dialysierflüssigkeit verläuft.

Zunächst sendet der erste Ultraschallwandler 29 einen Ultraschallimpuls schräg in Strömuugsrichtung, der von dem zweiten Wandler 30 empfangen und in ein elektrisches Signal umgewandelt wird, das die Rechen- und Auswerteirheit 35 zur Bestimmung des Dialysierflüssigkeitsflusses und der Zusammensetzung der Dialysierflüssigkeit auswertet. Daraufhin sendet der zweite Ultraschallwandler 30 einen Impuls schräg entgegen der Strömungsrichtung der Dialysierflüssigkeit, der von dem ersten Wandler 29 empfangen und in ein elektrisches Signal umgewandelt wird, das die Rechen- und Auswerteinheit 35 auswertet.

Die Auswertung der Impulse beruht auf den folgenden Umständen:

In Strömungsrichtung beträgt die Impulslaufzeit $t_1$:

$$t_1 = \frac{L}{c+w'}$$

c = Ausbreitungsgeschwindigkeit der Ultraschallimpulse

w = Strömungsgeschwindigkeit

w' = Strömungsgeschwindigkeit in der Ausbreitungsrichtung der Ultraschallimpulse

Entgegen der Fließrichtung beträgt die Impulslaufzeit $t_2$:

$$t_2 = \frac{L}{c-w'}$$

Löst man die beiden obigen Gleichungen nach c auf und setzt sie dann gleich, erhält man:

$$2w' = \frac{L}{t_1} - \frac{L}{t_2}$$

Über die trigonometrische Beziehung ergibt sich die Strömungsgeschwindigkeit w dann zu:

$$w = \frac{L}{2 \cos \alpha}\left(\frac{1}{t_1} - \frac{1}{t_2}\right)$$

w ist die mittlere Strömungsgeschwindigkeit längs des Schallstrahls. Zur Berechnung des Volumenstroms benötigt man aber die mittlere Geschwindigkeit $w_A$ über den Querschnitt A. $w_A$ erhält man, indem man w mit einem Profilbeiwert β multipliziert.

Der Dialysierflüssigkeitsfluß V berechnet sich dann nach der folgenden Gleichung:

$$V = A \bullet w_A = \beta \frac{L}{2 \cos \alpha}\left(\frac{1}{t_1} - \frac{1}{t_2}\right)A \qquad (1)$$

Die Rechen- und Auswerteinheit ermittelt aus den Ausgangssignalen der Ultraschallwandler 29, 30 die erste und zweite Impulslaufzeit $t_1$, $t_2$ und berechnet dann nach Gleichung (1) den Dialysierflüssigkeitsfluß, wobei die Länge L der Meßstrecke, der Winkel α und der Profilbeiwert β sowie die Querschnittsfläche A des Meßrohrs in der Rechen- und Auswerteinheit gespeichert sind.

Die Ausbreitungsgeschwindigkeit der Ultraschallimpulse ist der Dichte und damit der Konzentration direkt proportional. Die Rechen- und Auswerteinheit ermittelt die Ausbreitungsgeschwindigkeit c der Ultraschallimpulse in der Dialysierflüssigkeit nach folgender Gleichung:

$$c = \frac{L}{2}\left(\frac{1}{t_1} + \frac{1}{t_2}\right)$$

Die Ausbreitungsgeschwindigkeit c wird mit einem vorgegebenen Sollwert, der einer gewünschten Dialysierflüssigkeitszusammensetzung entspricht, in der Auswert- und Recheneinheit verglichen. Eine Abweichung von dem Sollwert wird der Steuereinheit 33 über die Datenleitung 25 mitgeteilt. Die Steuereinheit 33 steuert dann die Förderraten der Proportionierungspumpen 19, 20 so an, daß das Mischungsverhältnis wieder dem gewünschten Wert entspricht.

Wenn das dem Wasser beigemischte Konzentrat nur einen gelösten Stoff enthält, kann aus der Ausbreitungsgeschwindigkeit c der Ultraschallimpulse die der Ausbreitungsgeschwindigkeit proportionale Konzentration des gelösten Stoffes in der Dialysierflüssigkeit bestimmt werden.

Die Auswert- und Steuereinheit verfügt noch über eine Kompensationseinheit zur Kompensation der Abhängigkeit der Dichte von der Temperatur der Dialysierflüssigkeit, die mit dem Temperatursensor 12 gemessen wird.

Für den Fall, daß der Dialysierflüssigkeitsfluß V nicht von Interesse ist, können die beiden Ultraschallwandler 29, 30 auch derart angeordnet sein, daß die Meßstrecke in einem rechten Winkel zur Längsachse des Meßrohrs 28 verläuft ($\alpha = 90°$), wobei der erste Ultraschallwandler 29 Ultraschallimpulse aussendet, die der zweite Ultraschallwandler 30 empfängt. Der erste Ultraschallwandler ist also nur als Sender und der zweite Ultraschallwandler nur als Empfänger ausgebildet. Aus der Laufzeit der Ultraschallimpulse auf der Meßstrecke wird deren Ausbreitungsgeschwindigkeit bestimmt, die der Dichte und damit der Konzentration direkt proportional ist.

In Figur 3 ist die Änderung der Ausbreitungsgeschwindigkeit der Ultraschallimpulse in der Dialysierflüssigkeit als Funktion des Volumens bzw. der Menge von verschiedenen Substanzen (Graph I: Glucose; Graph II: Bicarbonat; Graph III: Säure) dargestellt, die der Dialysierflüssigkeit hinzugefügt wurden, wobei eine Temperaturkompensation nicht vorgenommen wurde. Während bei der bekannten Leitfähigkeitsmessung geringe Zugaben von Säurekonzentrat zur Dialysierflüssigkeit zu einem deutlichen Anstieg der elektrischen Leitfähigkeit, entsprechende Zugaben von Bicarbonatkonzentrat zur Dialysierflüssigkeit zu einem geringen Anstieg der elektrischen Leitfähigkeit und entsprechende Zugaben von Glucose zur Dialysierflüssigkeit zu keinem Anstieg der elektrischen Leitfähigkeit führen, führen geringe Zugaben von Säurekonzentrat oder Bicarbonatkonzentrat oder Glucose zur Dialysierflüssigkeit bei dem erfindungsgemäßen Meßverfahren zu vergleichbaren Anstiegen der ausgewerteten Ausbreitungsgeschwindigkeit der Ultraschallimpulse in der Dialysierflüssigkeit.

**Patentansprüche**

1. Einrichtung zur Bereitstellung von Dialysierflüssigkeit für eine Dialysevorrichtung, die eine Dialysierflüssigkeitsquelle (15 bis 21) und eine Einrichtung (24) zur Überwachung ausgewählter Parameter der Dialysierflüssigkeit aufweist,
dadurch gekennzeichnet,
daß die Einrichtung (24) zur Überwachung ausgewählter Parameter der Dialysierflüssigkeit aufweist:

    - Mittel (28) zur Aufnahme der Dialysierflüssigkeit,
    - Mittel (29, 30) zum Senden von Ultraschallimpulsen durch die Dialysierflüssigkeit,
    - Mittel (29, 30) zum Empfangen der Ultraschallimpulse,
    - Mittel (35) zum Ermitteln der Ausbreitungsgeschwindigkeit der Ultraschallimpulse in der Dialysierflüssigkeit und
    - Mittel (35) zum Bestimmen des ausgewählten Parameters aus der ermittelten Ausbreitungsgeschwindigkeit.

2. Vorrichtung nach Anspruch 1, dadurch gekennzeichnet, daß die Mittel zur Aufnahme der Dialysierflüssigkeit eine Meßleitung (28) umfassen, durch die die Dialysierflüssigkeit geleitet wird, und daß die Mittel zum Senden und die Mittel zum Empfangen der Ultraschallimpulse umfassen:
einen ersten Ultraschallwandler (29) zum Senden von Ultraschallimpulsen, der an dem einen Ende einer senkrecht zur Längsachse der Meßleitung verlaufenden Meßstrecke angeordnet ist und einen zweiten Ultraschallwandler zum Empfangen von Ultraschallimpulsen, der an dem anderen Ende der Meßstrecke angeordnet ist, wobei die Mittel (35) zum Bestimmen der ausgewählten Parameter derart ausgebildet sind, daß aus der Impulslaufzeit der Ultraschallimpulse längs der Meßstrecke als ausgewählter Parameter die Konzentration einer Substanz in der Dialysierflüssigkeit bestimmbar ist.

3. Vorrichtung nach Anspruch 2, dadurch gekennzeichnet, daß die Mittel zur Aufnahme der Dialysierflüssigkeit eine Meßleitung (28) umfassen, durch die die Dialysierflüssigkeit geleitet wird, und daß die Mittel zum Senden und die Mittel zum Empfangen der Ultraschallimpulse umfassen:

    - einen ersten Ultraschallwandler (29) zum Senden und Empfangen von Ultraschallimpulsen, der an dem einen Ende einer quer zur Längsachse der Meßleitung verlaufenden Meßstrecke angeordnet ist,
    - einen zweiten Ultraschallwandler (30) zum Senden und Empfangen von Ultraschallimpulsen, der an dem anderen Ende der Meßstrecke angeordnet ist und
    - eine Einrichtung (33) zum Umschalten des ersten und zweiten Ultraschallwandlers derart, daß zur Ermittlung einer ersten Impulslaufzeit ($t_1$) in Strömungsrichtung der durch die Meßleitung geleiteten Dialysierflüssigkeit der erste Ultraschallwandler (29) Ultraschallimpulse aussendet, die der zweite Ultraschallwandler (30) empfängt und zur Ermittlung einer zweiten Impulslaufzeit ($t_2$) entgegen der Strömungsrichtung der Dialysierflüssigkeit, der zweite Ultraschallwandler (30) Ultraschallimpulse aussendet, die der erste Ultraschallwandler (29) empfängt.

4. Vorrichtung nach Anspruch 3, dadurch gekennzeichnet, daß die Mittel (35) zum Bestimmen der ausgewählten Parameter derart ausgebildet sind, daß aus der ermittelten ersten und zweiten Impulslaufzeit ($t_1$, $t_2$) als ausgewählter Parameter der Dialysierflüssigkeitsfluß (V) bestimmbar ist.

5. Vorrichtung nach Anspruch 3, dadurch gekennzeichnet, daß die Mittel (35) zum Bestimmen der ausgewählten Parameter derart ausgebildet sind, daß aus der ermittelten ersten und zweiten Impulslaufzeit ($t_1$, $t_2$) als ausgewählter Parameter die Konzentration einer Substanz in der Dialysierflüssigkeit bestimmbar ist.

6. Vorrichtung nach einem der Ansprüche 1 bis 5, dadurch gekennzeichnet, daß die Mittel (35) zum Ermitteln der Ausbreitungsgeschwindigkeit (c) der Ultraschallimpulse in der Dialysierflüssigkeit eine Einrichtung zur Kompensation der Abhängigkeit der Ausbreitungsgeschwindigkeit von der Temperatur umfassen.

**7.** Verfahren zur Überwachung ausgewählter Parameter der Dialysierflüssigkeit bei einer Dialysebehandlung mit folgenden Verfahrensschritten:

- Senden von Ultraschallimpulsen durch die Dialysierflüssigkeit,
- Empfangen der Ultraschallimpulse,
- Ermitteln der Ausbreitungsgeschwindigkeit der Ultraschallimpulse in der Dialysierflüssigkeit und Bestimmen des ausgewählten Parameters aus der ermittelten Ausbreitungsgeschwindigkeit.

**8.** Verfahren nach Anspruch 7, dadurch gekennzeichnet, daß die Dialysierflüssigkeit durch eine Meßleitung geleitet wird, wobei die Ultraschallimpulse an einem Ende einer senkrecht zur Längsachse der Meßleitung verlaufenden Meßstrecke erzeugt und an dem anderen Ende der Meßstrecke zur Ermittlung der Impulslaufzeit längs der Meßstrecke empfangen werden und aus der ermittelten Impulslaufzeit als ausgewählter Parameter die Konzentration einer Substanz in der Dialysierflüssigkeit bestimmt wird.

**9.** Verfahren nach Anspruch 7, dadurch gekennzeichnet, daß die Dialysierflüssigkeit durch eine Meßleitung geleitet wird, wobei zur Ermittlung einer ersten Impulslaufzeit in Strömungsrichtung der durch die Meßleitung geleiteten Dialysierflüssigkeit Ultraschallimpulse an einem Ende einer quer zur Längsachse der Meßleitung verlaufenden Meßstrecke erzeugt und an dem anderen Ende der Meßstrecke empfangen werden und zur Ermittlung einer zweiten Impulslaufzeit entgegen der Strömungsrichtung der Dialysierflüssigkeit Ultraschallimpulse an dem anderen Ende der Meßstrecke erzeugt und an dem einen Ende der Meßstrecke empfangen werden.

**10.** Verfahren nach Anspruch 9, dadurch gekennzeichnet, daß aus der ermittelten ersten und zweiten Impulslaufzeit als ausgewählter Parameter der Dialysierflüssigkeitsfluß bestimmt wird.

**11.** Verfahren nach Anspruch 9, dadurch gekennzeichnet, daß aus der ermittelten ersten und zweiten Impulslaufzeit als ausgewählter Parameter die Konzentration einer Substanz in der Dialysierflüssigkeit bestimmt wird.

**12.** Verfahren nach einem der Ansprüche 7 bis 11, dadurch gekennzeichnet, daß die Abhängigkeit der Ausbreitungsgeschwindigkeit der Ultraschallimpulse in der Dialysierflüssigkeit von der Temperatur der Dialysierflüssigkeit kompensiert wird.

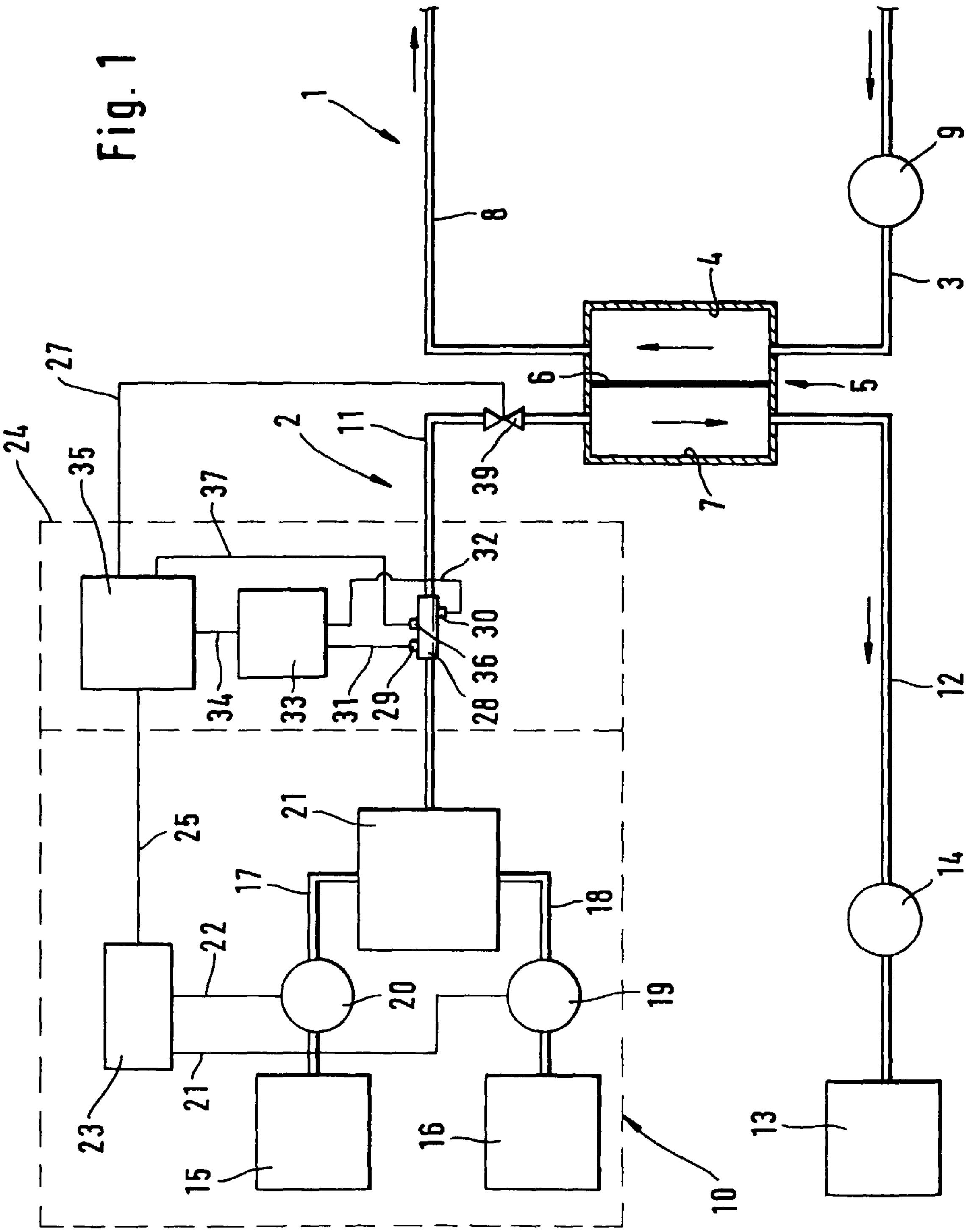

Fig. 1

Fig. 2

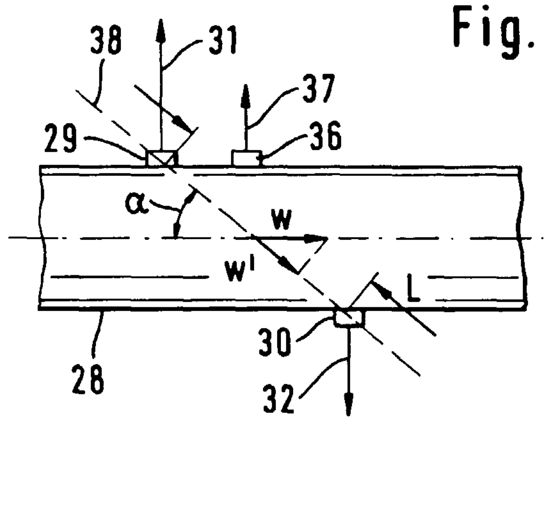
38
31
37
29
36
α
w
w'
L
30
28
32

Fig. 3

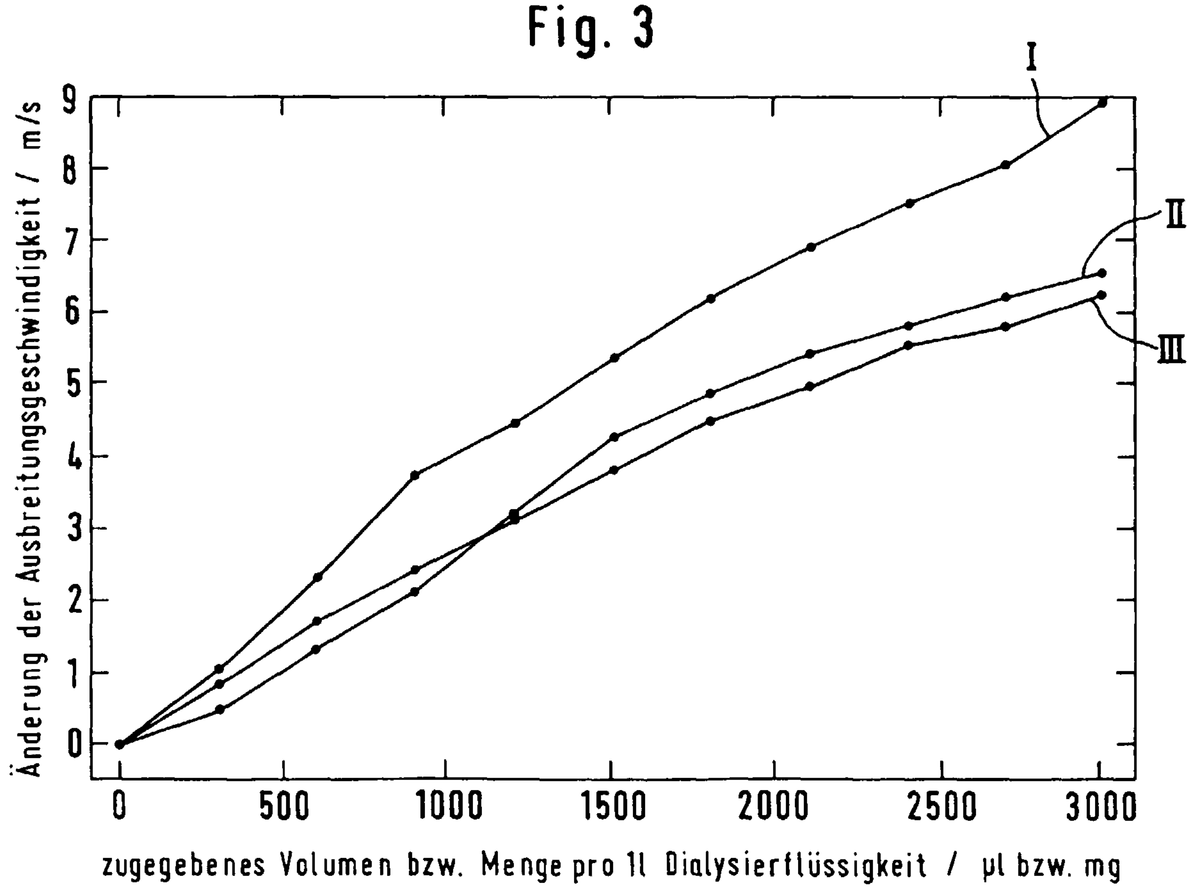
I
II
III
Änderung der Ausbreitungsgeschwindigkeit / m/s
0
1
2
3
4
5
6
7
8
9
0
500
1000
1500
2000
2500
3000
zugegebenes Volumen bzw. Menge pro 1l Dialysierflüssigkeit / µl bzw. mg

Europäisches Patentamt

# EUROPÄISCHER RECHERCHENBERICHT

Nummer der Anmeldung
EP 99 10 4453

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| X<br><br>Y | US 4 850 220 A (ASANO KIYOKAZU ET AL)<br>25. Juli 1989<br>* Spalte 2, Zeile 9-27 *<br>* Spalte 2, Zeile 50-55 *<br>* Spalte 3, Zeile 4 - Spalte 4, Zeile 62 *<br>* Spalte 7, Zeile 6-39; Abbildung 1 *<br>--- | 1,7<br><br>2-6,8-12 | B01F5/10 |
| Y | DE 38 27 553 C (FRESENIUS AG)<br>26. Oktober 1989<br>* Spalte 3, Zeile 25-34 *<br>* Abbildung 1 *<br>--- | 6,12 | |
| Y,D | US 4 015 470 A (MORRISON RODERICK G)<br>5. April 1977<br>* Spalte 2, Zeile 32-62 *<br>* Spalte 3, Zeile 14-63 *<br>* Spalte 820-24 *<br>* Spalte 13, Zeile 5 *<br>* Abbildungen 1,2 *<br>--- | 2-5,8-11 | |
| X | EP 0 243 547 A (PEABODY ALAN M)<br>4. November 1987<br>* Spalte 10, Zeile 9-42 *<br>* Abbildung 3 *<br>--- | 1,7 | RECHERCHIERTE SACHGEBIETE (Int.Cl.6)<br>A61M<br>G01F<br>G01H<br>B01F |
| X | PATENT ABSTRACTS OF JAPAN<br>vol. 013, no. 562 (C-665),<br>13. Dezember 1989<br>& JP 01 232970 A (MISHIMA WAAKU KK),<br>18. September 1989<br>* Zusammenfassung *<br>--- | 1,7 | |
| A | DE 32 23 051 A (FRESENIUS AG)<br>29. Dezember 1983<br>* das ganze Dokument *<br>---<br>-/-- | 1,7 | |

Der vorliegende Recherchenbericht wurde für alle Patentansprüche erstellt

| Recherchenort | Abschlußdatum der Recherche | Prüfer |
|---|---|---|
| MÜNCHEN | 28. Juni 1999 | Bichlmayer, K-P |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

Europäisches Patentamt

# EUROPÄISCHER RECHERCHENBERICHT

Nummer der Anmeldung
EP 99 10 4453

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| A | EP 0 619 135 A (S I F RA SOCIETA ITALIANA FARM) 12. Oktober 1994<br>* Spalte 6, Zeile 38-51 *<br>* Spalte 7, Zeile 26-28 *<br>* Abbildungen 1,7 *<br>----- | | |

RECHERCHIERTE SACHGEBIETE (Int.Cl.6)

Der vorliegende Recherchenbericht wurde für alle Patentansprüche erstellt

| Recherchenort | Abschlußdatum der Recherche | Prüfer |
|---|---|---|
| MÜNCHEN | 28. Juni 1999 | Bichlmayer, K-P |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR. EP 99 10 4453

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

28-06-1999

| Im Recherchenbericht angeführtes Patentdokument | Datum der Veröffentlichung | Mitglied(er) der Patentfamilie | Datum der Veröffentlichung |
|---|---|---|---|
| US 4850220 A | 25-07-1989 | JP 2556701 B | 20-11-1996 |
| | | JP 63286162 A | 22-11-1988 |
| DE 3827553 C | 26-10-1989 | AT 106756 T | 15-06-1994 |
| | | DE 58907818 D | 14-07-1994 |
| | | EP 0358873 A | 21-03-1990 |
| | | ES 2057028 T | 16-10-1994 |
| | | JP 2211173 A | 22-08-1990 |
| | | JP 2869735 B | 10-03-1999 |
| | | US 5230341 A | 27-07-1993 |
| US 4015470 A | 05-04-1977 | KEINE | |
| EP 0243547 A | 04-11-1987 | AT 65416 T | 15-08-1991 |
| | | US 4586920 A | 06-05-1986 |
| DE 3223051 A | 29-12-1983 | AT 36963 T | 15-09-1988 |
| | | DE 3377897 A | 13-10-1988 |
| | | EP 0097366 A | 04-01-1984 |
| | | JP 1592778 C | 14-12-1990 |
| | | JP 2014853 B | 10-04-1990 |
| | | JP 59057662 A | 03-04-1984 |
| | | US 4508622 A | 02-04-1985 |
| EP 0619135 A | 12-10-1994 | IT 1260638 B | 22-04-1996 |
| | | CA 2120593 A | 06-10-1994 |

EPO FORM P0461

Für nähere Einzelheiten zu diesem Anhang : siehe Amtsblatt des Europäischen Patentamts, Nr.12/82